# EUROPEAN PATENT SPECIFICATION

(11) **EP 2 995 270 B1**
(45) Date of publication and mention of the grant of the patent: **14.02.2018**
(21) Application number: 15460064.7
(22) Date of filing: 03.09.2015
(51) Int. Cl.: A61B 17/80, A61B 17/86

(54) **THE SET OF BONE PLATE AND BONE SCREW USED TO STABILIZE FRACTURES**
SATZ VON KNOCHENPLATTE UND KNOCHENSCHRAUBE ZUR STABILISIERUNG VON FRAKTUREN
ENSEMBLE DE PLAQUES OSSEUSES ET VIS À OS UTILISÉS POUR STABILISER DES FRACTURES

(30) Priority: 12.09.2014 PL 40947614
(43) Date of publication of application: 16.03.2016
(73) Proprietor: "CHM" sp. z o.o., 16-061 Juchnowiec (PL)
(72) Inventor: Marczynski, Wojciech Józef, 02-849 Warszawa (PL); Ratynski, Grzegorz Maria, 05-804 Pruszków (PL); Sobolewski, Andrzej, 15-057 Bialystok (PL); Krzyzek, Andrzej, 15-666 Bialystok (PL); Charkiewicz, Marcin Pawel, 15-077 Bialystok (PL)

(56) References cited:
- JP-A- H01 300 945
- US-A1- 2002 058 940
- US-A1- 2008 200 955
- US-A1- 2010 016 858
- US-A1- 2013 150 853
- US-A1- 2013 211 461

## Description

The invention relates to the set of bone plate and bone screw used to stabilize periarticular and/or shaft fractures of the skeletal system.

In clinical treatment of bone fractures with internal stabilization using bone plates, there are many structural embodiments used for periarticular and/or shaft fractures stabilization based on the use of bone plate and locking screws. In this treatment, the bone union relies on a stable connection of a plate with a locking screw which allows for angular stable fixation of bone fragments, preventing the fragments and bone screws from migration. In orthopedics, the most widely used connection is a threaded connection where a locking screw has a threaded head that cooperates with a threaded hole of a bone plate.

Complex shapes of locking plates, physiological limitations referring to the plates thickness and angular adjustment of the screws that are forced by a complex anatomy of the human skeleton, pose problems in use of the currently offered structural embodiments, in particular, the heads of the locking screws that protrude above the top or bottom surface of the plate which causes i.a. irritation and compression of peri-implant tissues (tendons, skin, etc.). The problem also relates to a limited number of active thread coils of the locking plate hole, resulting in lower mechanical strength of the screw-plate threaded connection. Moreover, in the current state of the art, there is a limited range of angular positioning of the screw in relation to the plate surface. Overgrowing of the exposed portion of the thread with tissues impedes the subsequent removal of the implant.

In the current state of the art the above-mentioned problems are solved by:
- making the plate thicker which, however, is not applicable in all types of fracture fixation,
- reducing the height of the screw head, consequently reducing the cooperating surface of the plate-screw construct, thus lowering the mechanical strength of the connection,
- performing conical extrusions applicable to thin bone plates of thickness of 0.5±2mm. This embodiment has a great limitation regarding the angle of the screw inclination in relation to the plate surface - the need to maintain concentricity of the extrusion and the axis of the locking hole.

The current state of the art relating to the set used to stabilize periarticular and/or shaft fractures of the skeletal system is described i.a. in the Patents numbered: EP1211992 B1, EP1211993 B1, EP1610700 B1, EP2040631B1, US6821278B2, EP1865866 B1 (US 2008/200955 A1) and in the Inventions Applications numbered: EP2559394 A1 and US 2004/0153073 A1, US 2002/058940 A1 (EP 1294298 B1), JP H01 300945 A.

The European Patent EP1211992 B1 discloses an embodiment where a conical threaded hole is performed in the bone plate of the set that comprises at least one locking screw mounted to the plate in a fixed angular relationship. However, in this embodiment, when larger angles of the locking hole axis inclination occur, the screw will not work with its entire perimeter with the plate hole and the screw head will protrude above the surface of the plate. A part of the locking screw thread will be exposed and the peri-implant tissue overgrowing may occur.

The European Patent EP1211993 B1 discloses an embodiment where a hole is performed with an element that gets deformed during screw insertion, without locking thread in the hole of the plate. This allows the insertion of the screw at a different angle, however, threaded screw head works with a locking hole of a plate at a very short distance. In this embodiment, the locking thread is exposed and the peri-implant tissue overgrowing may occur.

The embodiment presented in European Patent EP1610700 B1 refers to two adjacent threaded locking holes with the possibility to make a hole at an angle. The top recess allows the screw head to immerse. A similar screw embodiment as ChLP screw causes protrusion of the screw at the higher inclination angles and in thin plates. The locking threads are exposed and the peri-implant tissue overgrowing may occur.

The next European Patent - EP2040631 B1 discloses an embodiment where a calcaneal plate with local thickened cross-section was presented. This allows for positioning of the screw at a greater angle. The local increase in thickness of the plate is not always possible due to the anatomical and technical limitations of the device.

In the European Invention Application - EP2559394 A1 - the embodiment described presents a locking hole and screws with the possibility of changing the angle of insertion. Half-thread profiles may weaken screw-plate connection. Although performed upper deepening eliminates the screw head protrusion above the top surface of the plate, this embodiment is limited by the thickness of the plate. When angularly positioned, locking threads of the screw head are exposed and the peri-implant tissue overgrowing may occur.

The American Invention Application US 2004/0153073 A1 presents locking using threaded end cap. The disadvantage of this embodiment is introduction of an additional locking element. Moreover, the connection strength is lower than in the case of a direct threaded screw-plate connection.

The American Patent US6821278B2 discloses an embodiment where a plate with conical bends and locking holes in them is presented. This embodiment limits the inclination angle of the screw in relation to the plate surface. It does not eliminate, however, the protrusion of the screw head above the top surface of the plate.

In the American Patent description US 2002/058940 A1 (EP1294298 B1) the embodiment described presents a plate set of a thickness of mainly 0.5÷2 mm with a screw, having a conical projection and a corresponding conical cavity on the opposite side of the plate. Insertion of the bone screw at an angle in relation to the surface of the plate results in the increased screw head protrusion above the surface of the bone plate, and incomplete coverage of the thread of the bone screw head.

The Japanese Invention Application JP H01 300 945 discloses an embodiment where two or more thin plates are placed one on the other, wherein one of the plates has projections corresponding to the holes in the other plate. This embodiment includes non-locking, conventional bone screws. The said embodiment does not solve the problems that occur when inserting locking screws with threaded head at an angle. Application of the cited embodiment, with the angular positioning of the screw with a threaded head, would result in exposing the part of the thread in the upper or lower surface thereof.

The aim of this invention is the embodiment relating to the set used to stabilize periarticular and/or shaft fractures of the skeletal system wherein the screw head, when angularly positioned in relation to the plate, does not protrude over the top surface of the plate. Furthermore, the locking thread of the screw should not protrude beyond the bottom convex portion of the bulge and the bottom surface of the plate.

The invention relates to the set of bone plate and bone screw used to stabilize fractures, wherein the angularly positioned locking screw comprises threaded head, and the bone plate comprises a convex shaped surface, wherein the threaded head of the bone screw cooperates with a threaded hole located in the place of that surface of the bone plate, characterized in that the plate in the area of holes for bone screws insertion has a bulge irrespective of (not relating to and not form-fitting with) the deepening and which is of a spherical zone shape located on the top and/or the bottom surface.

The bottom bulge comprises a recess perpendicular to the axis of the plate hole, and in the plate bulge surrounding, the plate top and bottom surfaces are parallel.

Regardless of the angle of inclination α of the plate hole axis in relation to the axis perpendicular to the top surface of the plate, the screw head is always flush with or below the top surface of the plate and the locking screw thread is flush with or above the bottom surface of the plate.

In the plate that has one spherical zone, there is a deepening located on the top or bottom surface of the plate in the axis of the hole.

In an advantageous embodiment, the depth in the deepening in the axis of the hole in relation to bottom or top surfaces of the plate increases with the angle of inclination α of the plate hole axis in relation to the axis perpendicular to the top surface of the plate.

The embodiment of the set used to stabilize periarticular and/or shaft fractures of the skeletal system eliminates the most common problems in the current state of the art. The shape of the plate surface in the region of holes for bone screws insertion allows for the optimum thickness of the plate without its thickening on the entire length. Applied bulges in the shape of a spherical zone located in the top and/or the bottom surface do not limit the inclination angle of the screw in relation to the plate surface and do not enforce the need to maintain concentricity of the bulge and the locking hole axis.

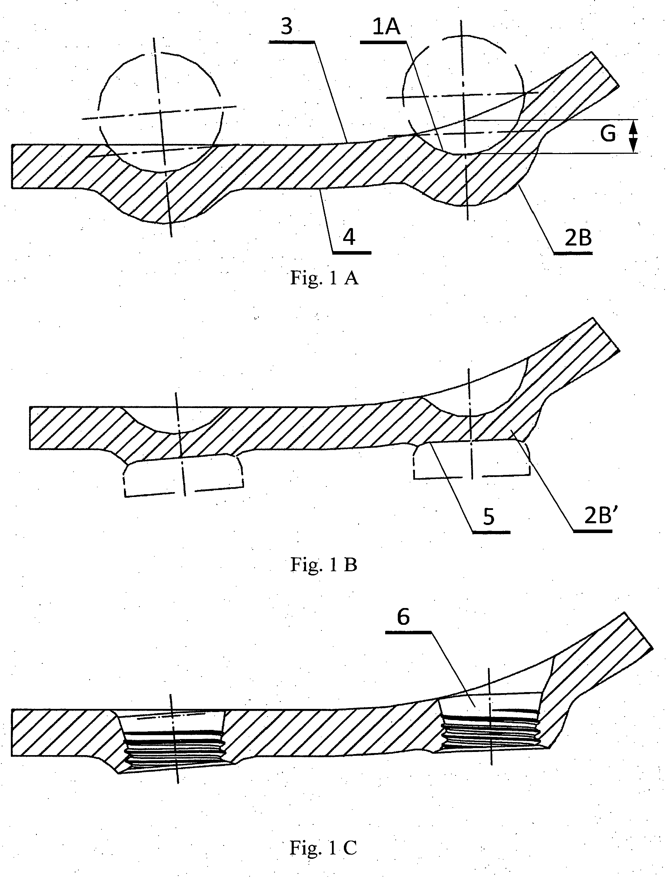

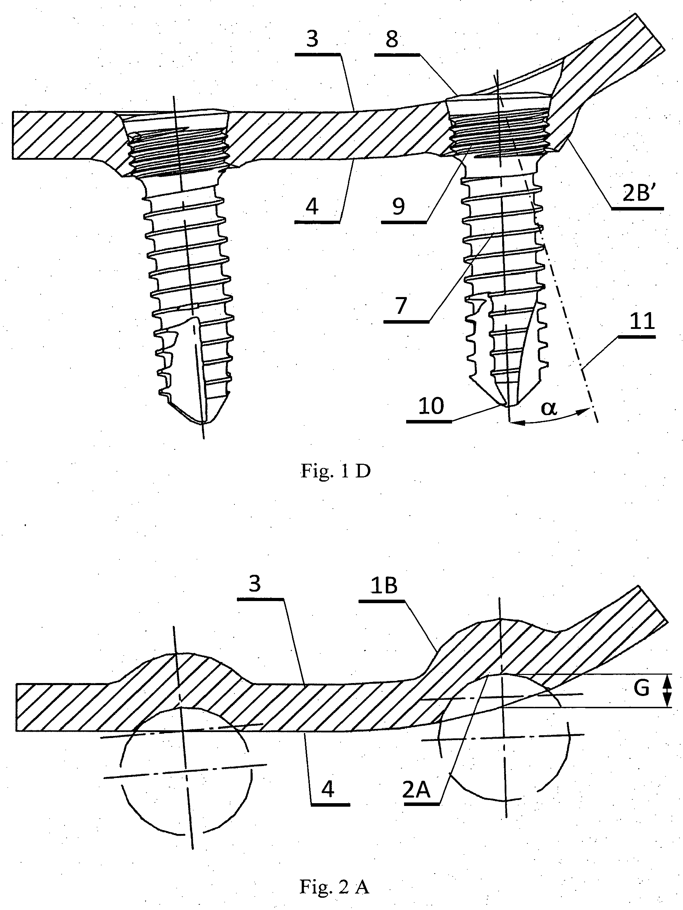

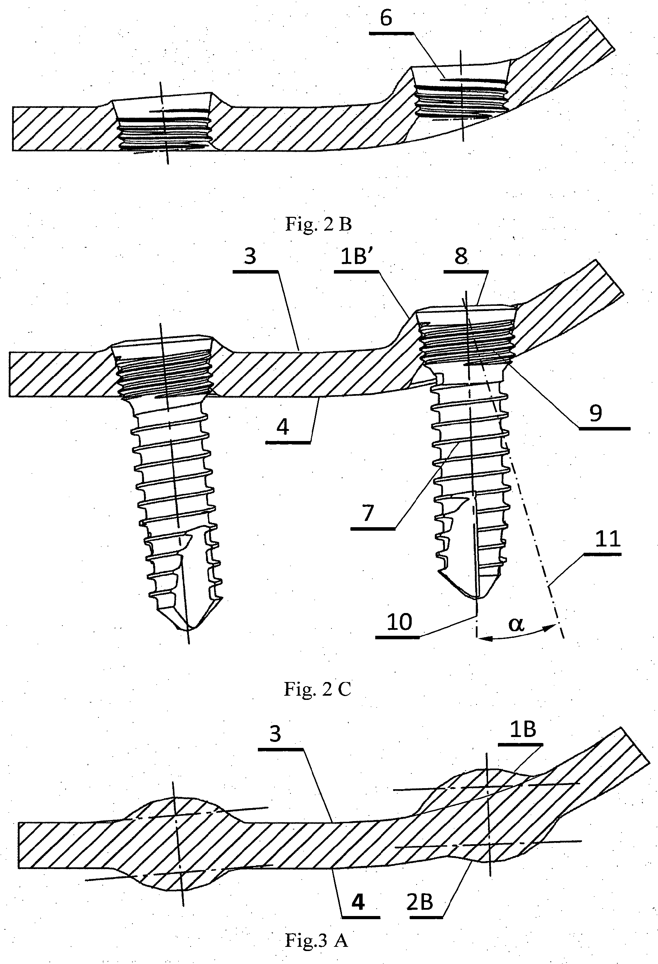

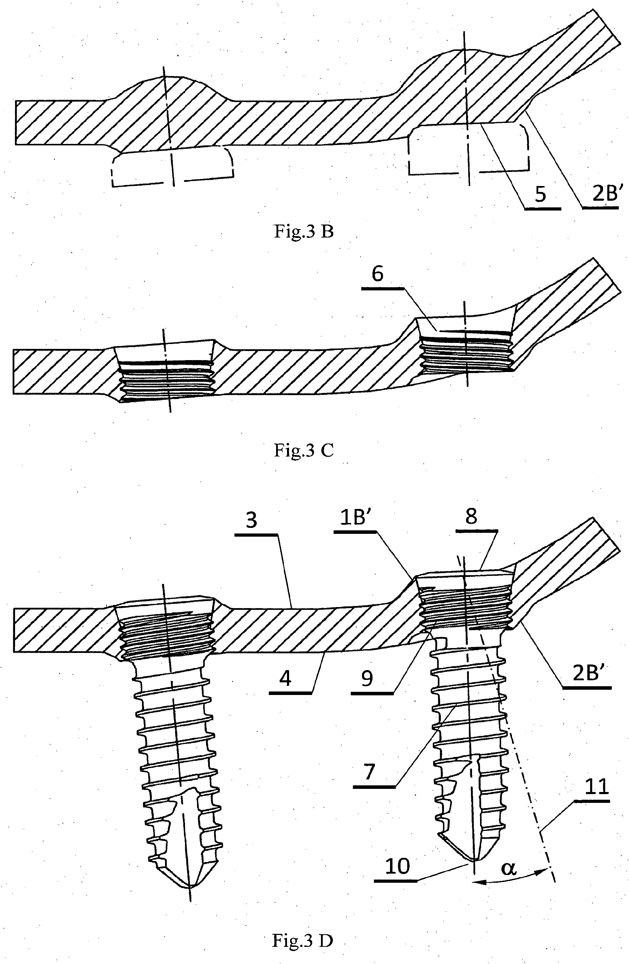

The invention is presented for example on a drawing in which: Fig. 1A illustrates a bone plate with a bulge having a concave portion in relation to the top surface of the plate and the convex portion in relation to the bottom surface of the plate; Fig. 1B shows a bone plate with a recess in the bottom convex portion of the bulge of a spherical zone shape; Fig. 1C describes a bone plate with a hole for a bone screw insertion formed in the bulge; Fig. 1D presents the set used to stabilize periarticular and/or shaft fractures of the skeletal system where the screw is angularly positioned in relation to the plate; Fig. 2A depicts a plate with the bulge having a convex portion in relation to the top surface of the plate, and a concave portion with respect to its bottom surface; Fig. 2B illustrates a bone plate with a hole for a bone screw insertion in the bulge that has a convex portion in relation to the top surface of the plate and a concave portion as a deepening with respect to its bottom surface; Fig. 2C shows the set used to stabilize periarticular and/or shaft fractures of the skeletal system where in the angular position of the screw with respect to the plate, the screw head does not protrude above the top surface of the plate and its convex portion; Fig. 3A describes a bone plate with the convex bulge on its bottom and top part; Fig. 3B presents a bone plate with cylindrical or conical recesses in the bottom convex part of the bulge; Fig. 3C depicts a bone plate of the set with the hole for screw insertion performed in the bulge; Fig. 3D illustrates the set where in the angular position of the screw with respect to the plate, the screw head does not protrude above the top surface of the plate and convex portion of the bulge spherical zone, and the locking thread of the screw does not extend beyond the bottom portion of the bulge spherical zone and the bottom surface of the plate.

The set used to stabilize periarticular and/or shaft fractures of the skeletal system consists of a bone plate 1 and bone screw 7.

In the sets provided below, the appropriate configurations of the plate surface in the region of the holes 6 for bone screws insertion 7 are introduced. The embodiment shown in the drawing in Fig. 1A ÷ Fig. 1C shows a bone plate of the set with a shaped bulge that has a deepening 1A in relation to the top surface 3 of the plate, and a convex portion 2B with respect to the bottom surface 4 of the plate. In the bottom portion of the bulge 2B, a cylindrical or conical recess 5 which is perpendicular to the axis 10 of the bone plate 1 hole 6 is performed as presented on Fig 1B and 3B. Fig. 1D, 3D show the bulge bone plate set 1 with a hole 6 in it for the appropriate bone screw 7 insertion and bulge of a spherical zone shape 2B' or 1B'. When the screw 7 is positioned angularly with respect to the plate 1, the screw 7 head 8 does not protrude above the top surface 3 of the bulge of a spherical zone shape 1B' of the plate 1 and the screw 7 locking thread 9 does not protrude beyond the bottom convex portion of the bulge of a spherical zone shape 2B' and the bottom surface 4 of the plate 1. The depth G of the deepening 1A and 2A in the axis of the hole 6 as measured from the top 3 or bottom 4 surface of the plate 1 is dependent on the angle α of inclination of the plate 1 hole 6 axis 10 with respect to the axis 11 perpendicular to the top surface 3 as shown in Fig. 1D.

The recess 5 performed in the bulge smoothes the edge resulted from the hole 6 performed. Additionally, convex portion 2B of the bulge forces the bottom surface 4 of the plate 1 to be pulled back in relation to the bone surface which results in a greatly reduced plate-bone contact surface which further on preferably influences peri-implant blood circulation and accelerates bone healing. Furthermore, the head 8 of the locking screw 7 works with its entire perimeter with the hole 6.

Fig. 2A÷Fig.2C show the embodiment with a shaped, i.e. spherical or oval-shaped bulge that has a convex portion 1B with respect to the top surface 3 of the plate 1 and the concave portion 2A (deepening) in relation to its bottom surface 4. In the bulge, a hole 6 for a suitable bone screw insertion was performed and when the screw 7 is positioned angularly in relation to the plate 1, the head 8 of the screw 7 does not protrude above the top surface 3 of the plate 1 and a convex portion 1B of the bulge and the locking thread 9 of the screw 7 does not protrude beyond the bottom surface 4 of the plate 1. Furthermore, the head 8 of the screw 7 works with its entire perimeter with the hole 6. As shown in Fig. 3A+Fig.3D, the bone plate 1 has a spherical or oval-shaped bulges that have a convex portion 1B with respect to the top surface 3 of the plate 1, and a convex portion 2B in relation to the bottom surface 4 of the plate 1, as shown in Fig. 3A. Additionally, in the bottom portion of the convex 2B of the bulge, a cylindrical or conical recess 5 is performed as shown in Fig. 3B. In that bulge, a hole 6 is performed for appropriate screw insertion. Similarly to two previous embodiments, when the screw 7 is angularly positioned in relation to the plate 1, the screw 7 head 8 does not protrude above the top surface 1B of the bulge and a convex portion 2B of the bulge, and the locking thread 9 of the screw 7 does not protrude beyond the bottom portion of the convex 2B of the bulge and the bottom surface 4 of the plate 1, as presented on Fig 3D. The depth of the bulge in the shape of a spherical zone 1B', 2B' is dependent on the angle α of inclination of the plate 1 hole 6 axis 10 with respect to the axis 11 perpendicular to the top surface 3 of the plate 1. The recess 5 performed in the bulge smoothes the edge resulted from the hole 6 performed. Additionally, convex portion 2B of the bulge forces the bottom surface 4 of the plate 1 to be pulled back in relation to the bone surface which results in a greatly reduced plate-bone contact surface which further on preferably influences peri-implant tissue blood circulation and accelerates bone healing. Furthermore, the head 8 of the screw 7 works with its entire perimeter with the hole 6.

## Claims

1. The set of bone plate (1) and bone screw (7) used to stabilize fractures, wherein the bone screw (7) is an angularly positioned locking screw comprising a threaded head (8) and the bone plate (1) comprises a convex shaped surface (1B, 2B) wherein the threaded head (8) of the bone screw (7) cooperates with a threaded hole located in the place of that surface (1B, 2B) of the bone plate (1) **characterized in that** the plate (1) in the area of holes (6) for bone screws (7) insertion has at least one bulge (1B, 2B) in relation to the top (3) and/ or bottom (4) surface of the plate (1), irrespective of (not relating to and not form-fitting with) the deepening (1A, 2A), whereas the bulge (1B, 2B) of the plate (1) in the area of holes (6) for bone screws (7) insertion is of a spherical zone shape (1B', 2B') located on the top surface (3) and/or the bottom surface (4), and in the bulge (1B, 2B) of the plate (1) surrounding, the top (3) surface and the bottom (4) surface of the plate (1) are parallel in relation to each other, and the head (8) of the screw (7) is always flush with or below the top (3) surface of the plate (1) and the locking thread (9) of the screw (7) is flush with or above the bottom (4) surface of the plate (1), providing stable angular locking of the screw (7) in the plate (1).

2. The set acc. to claim 1 **characterized in that** in the plate (1) that has one spherical zone (1B', 2B'), there is a deepening (1A, 2A) located on the top (3) or bottom (4) surface of the plate (1) in the axis of the hole (6) whose depth (G) in the axis of the hole (6) in relation to surfaces (3,4) of the plate (1) increases with the angle of inclination (α) of the plate (1) hole (6) axis (10) in relation to the axis (11) perpendicular to the top (3) surface of the plate (1).

3. The set acc. to claim 1 **characterized in that** the bulge (2B) has a recess (5) that is perpendicular to the axis (10) of the hole (6) of the plate (1).

## Patentansprüche

1. Knochenplattenset (1) mit Knochenschraube (7) zur Stabilisierung der Brüche, in dem die Knochenschraube eine winkelig angeordnete Schraube mit einem Gewindekopf (7) ist, und die Knochenplatte (1) eine konvexe Formfläche hat (1B, 2B), in der der Gewindekopf (8) der Knochenschraube (7) mit einer Gewindebohrung im dieser Fläche (1B, 2B) der Knochenplatte (1) zusammenwirkt, **gekennzeichnet dadurch, dass** die Platte (1) n der Gegend der Bohrungen (6) für Knochenschrauben (7) mindestens eine Ausbauchung (1B,2B) gegenüber der oberen (3) oder unteren Fläche (4) hat, die unabhängig von der Vertiefung (1A, 2A), (nicht verbunden und nicht formableitend) ist, wobei die Ausbauchung (1B, 2B) der Platte (1) in der Gegend der Bohrung (6) für Knochenschrauben (7) die Gestalt eines kugelartigen Streifens (1B', 2B') auf der oberen (3) und/ oder unteren Fläche hat (4), und in der Gegend der Ausbauchung (1B, 2B) der Platte (1) i obere (3) und untere (4) Fläche (1) parallel gegenüber sind, der Kopf (8) der Schraube (7) befindet sich dagegen immer bündig mit der oberen Fläche (3) der Platte (1) oder etwas tiefer, und das blockierende Gewinde (9) der Schraube (7) immer bündig mit der unteren Fläche (4) der Platte (1) oder etwas tiefer und verursacht dadurch stabile, winkelige Blockierung der Schraube (7) in der Platte (1)."

2. Ein Satz gemäß Anspruch 1 **gekennzeichnet dadurch, dass** in der Platte (1) die einen kugelartigen Streifen hat (1B', 2B') auf der oberen Fläche (3) oder unteren (4) Fläche (1) in der Achse der Bohrung (6) eine Vertiefung existiert (1A, 2A), deren Tiefe (G) in der Achse der Bohrung (6) von der Oberfläche (3, 4) der Platte (1) desto größer ist, je größer der Abneigwinkel (α) der Achse (10) der Bohrung (6) von Platte (1) gegenüber der Achse (11) die zur oberen (3) Fläche rechtwinklig verläuft (1) ist.

3. Ein Satz gemäß Anspruch 1 **gekennzeichnet dadurch, dass** die Ausbauchung (2B) eine Nut hat (5), die senkrecht zur Achse (10) der Bohrung (6) von Platte (1) verläuft.

## Revendications

1. Implant d'ostéosynthèse constitué d'une plaque (1) et de vis (7) où l'angle de la vis d'ostéosynthèse est réglé à l'aide d'une vis verrouillable à tête filetée (8). La surface de la plaque (1) est convexe (1B, 2B). La tête filetée (8) de la vis d'ostéosynthèse (7) fonctionne avec un alésage taraudé situé en surface (1b, 2b) de la plaque (1) et est **caractérisé par le fait que** la plaque (1) dans la zone des ouvertures (6) destinées aux vis d'ostéosynthèse (7) a au moins un renflement (1B, 2B) par rapport à la surface supérieure (3) ou inférieure (4) indépendantes (non liées et sans rapport de forme) à la partie en creux (1A, 2A). Le renflement (1B, 2B) de la plaque (1) dans la zone des ouvertures (6) des vis d'ostéosynthèse (7) se présente sous la forme d'un segment de sphère (1B' 2B') en surface supérieure (3) et/ou inférieure (4). Dans la zone de renflement (1B, 2B) de la plaque (1) les surfaces supérieures (3) et inférieures (4) sont parallèles entre elles. La tête de vis (8) de la vis (7) se trouve toujours au niveau ou sous la surface supérieure (3) de la plaque (1) et le filetage bloquant (9) de la vis (7) se trouve au niveau ou en dessous de la surface inférieure (4) de la plaque (1) assurant ainsi la stabilité de la position angulaire de la vis (7) dans la plaque (1).

2. Selon la revendication 1, l'ensemble se **caractérise par le fait que**, sur la plaque (1) dotée d'un segment sphérique (1B', 2B') en partie supérieure (3) ou inférieure (4), se trouve une partie en creux (1A, 2A) dans l'axe de l'ouverture (6) et dont la profondeur (G) mesurée dans l'axe de l'ouverture (6) à partir des surfaces (3, 4) de la plaque (1) est d'autant plus grande que la déclinaison angulaire (α) de l'axe (10) de l'ouverture (6) de la plaque (1) par rapport à l'axe (11), perpendiculaire à la surface supérieure (3), est important.

3. Selon la revendication 1, l'ensemble se **caractérise par le fait que** le renflement (2b) présente un évidement (5) perpendiculaire à l'axe (10) de l'ouverture (6) de la plaque (1).
